(19) Europäisches Patentamt
European Patent Office
Office européen des brevets

(11) Publication number: 0 414 931 B1

# (12) EUROPEAN PATENT SPECIFICATION

(45) Date of publication of patent specification: 14.07.93 (51) Int. Cl.$^5$: A61K 31/415

(21) Application number: 89115881.8

(22) Date of filing: 29.08.89

## (54) Hypotensive agent.

(43) Date of publication of application:
06.03.91 Bulletin 91/10

(45) Publication of the grant of the patent:
14.07.93 Bulletin 93/28

(84) Designated Contracting States:
AT BE CH DE FR GB IT LI LU NL SE

(56) References cited:
EP-A- 0 268 800

W.Forth et al., ALLGEMEINE UND SPEZIELLE PHARMAKOLOGIE UND TOXIKOLOGIE, 1977; pp. 122-126&NUM;

(73) Proprietor: TANABE SEIYAKU CO., LTD.
2-10, Dosho-machi 3-chome
Chuo-ku Osaka(JP)

(72) Inventor: Takamura, Norio
No. 20-6, Shinshiraoka 2-chome Shiraoka-machi
Minamisaitama-gun Saitama-ken(JP)
Inventor: Oda, Kuniyuki
No. 23-14, Ryoke 3-chome
Urawa-shi Saitama-ken(JP)
Inventor: Kodato, Shin-ichi
Green Park Nishikawaguchi 206 No. 18-11, Naka-cho
Kawaguchi-shi Saitama-ken(JP)
Inventor: Yamaguchi, Isao
No. 3-16-8, Ogikubo Suginami-ku
Tokyo-to(JP)
Inventor: Yano, Koji
No. 5-72-307, Hibarigaoka 3-chome
Hoya-shi Tokyo-to(JP)

(74) Representative: Kraus, Walter, Dr. et al
Patentanwälte Kraus, Weisert & Partner
Thomas-Wimmer-Ring 15
W-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of a phenoxyacetic acid derivative of the formula (I):

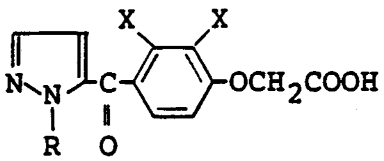

(I)

wherein R is a $C_1$-$C_6$-alkyl group or a $C_1$-$C_6$-alkoxy-substituted $C_1$-$C_6$-alkyl group and X is a halogen atom, or a pharmaceutically acceptable salt thereof for the manufacture of a hypotensive agent.

Hypertension is a disease showing the rising of blood pressure due to the increase of resistance of peripheral resistance vessel accompanied to functional or organic change, and is generally divided into two categories, i.e. essential hypertension and secondary hypertension according to the origin thereof. Hitherto, there have been known hypotensive agents which are used in the treatment of these hypertensions, such as vasodilators (e.g. hydralazine, etc.), adrenoceptor blockers including α-blockers (e.g. prazosin, etc.), and β-blockers (e.g. pindrol, etc.), angiotensin converting enzyme inhibitors (e.g. captopril, etc.), calcium antagonists (e.g. nifedipine, etc.), and diuretics (e.g. hydrochlorothiazide, etc.).

EP-A-0 268 800 discloses a diuretic pharmaceutical composition comprising a phenoxyacetic derivative of the formula (I) or a salt thereof and a pharmaceutically acceptable carrier therefor. Nothing can be found in this reference that the compounds are also valuable for the manufacture of a hypotensive agent.

The phenoxyacetic acid derivatives (I) and pharmaceutically acceptable salts thereof, which are the active ingredient for use in the preparation of the hypotensive agent have an excellent hypotensive activity. For instance, when the phenoxyacetic acid derivative was administered orally once at a dose of 200 mg/kg to spontaneously hypertensive rats (hereinafter referred to as SHR, about 40 weeks old) fasted overnight and the hypotensive activity thereof was estimated, the systolic blood pressure of the rats in the test compound-administered group, wherein [2,3-dichloro-4-(1-ethoxymethyl-5-pyrazolylcarbonyl)phenoxy]acetic acid and [2,3-dichloro-4(1-ethyl-5-pyrazolylcarbonyl)phenoxy]acetic acid were used as the active ingredient, showed a decrease of about 13 % 2 hours after administration in comparison with that of before administration.

The suitable examples of the phenoxyacetic acid derivatives useful as the active ingredient in the hypotensive agent are the compounds of the formula (I) wherein X is a halogen atom such as chlorine or bromine atom, and among them, preferable compounds are the compounds of the formula (I) wherein R is an alkyl group of 1 to 4 carbon atoms or an alkyl group of 1 to 3 carbon atoms substituted by an alkoxy group of 1 to 3 carbon atoms, and X is chlorine atom.

Particularly preferable compounds are the compounds of the formula (I) wherein R is an ethyl group or an ethoxymethyl group and X is chlorine atom, more specifically the following compounds:

[2,3-dichloro-4-(1-ethoxymethyl-5-pyrazolylcarbonyl)phenoxy]acetic acid

[2,3-dichloro-4-(1-ethyl-5-pyrazolylcarbonyl)phenoxy]acetic acid.

The phenoxyacetic acid derivatives (I) and the pharmaceutically acceptable salts thereof show an excellent hypotensive activity and hence, can be used in the treatment and prophylaxis of essential hypertension and various secondary hypertensions such as renal hypertension, endocrine hypertension, cardiovascular hypertension, hypertension accompanied to pregnancy, hypertension accompanied to acute stress, hypertension due to drugs, and alcoholic hypertension and the other hypertensions.

The phenoxyacetic acid derivatives (I) can be used either in the free form or in the form of a pharmaceutically acceptable salt thereof, and suitable examples of the pharmaceutically acceptable salt of the compound (I) are alkali metal salts (e.g. sodium salt or potassium salt), alkaline earth metal salts (e.g. calcium salt), inorganic acid salts (e.g. hydrochloride or hydrobromide) and organic acid salts (e.g. methanesulfonate or oxalate).

The hypotensive agent can be administered either orally or parenterally. For oral administration, the hypotensive agent can be used in the form of a pharmaceutical preparation which may contain pharmaceutical carriers suitable for oral administration such as vehicles, binders, disintegrators and wetting agents. The pharmaceutical carriers include, for example, starch, lactose, glucose, gelatin, sorbitol, tragacanth, polyvinylpyrrolidone, sugar, corn starch, polyethylene glycol, talc, potassium phosphate, magnesium stearate, and the other conventional vehicles, binders or disintegrators and wetting agents. The pharmaceutical preparation may be used in the solid dosage form such as tablets, powders, capsules, granules and

the like or in the liquid dosage form such as solutions, suspensions, emulsions, syrups or elixirs. Moreover, when administrated parenterally, the pharmaceutical preparation is preferably used in the form of injections. Suitable examples of solvents for the preparation for injections are distilled water for injection, vegetable oil or propylene glycol. Further, the pharmaceutical preparation for injections may contain safe solubilizers, buffer agents, stabilizers, and the like.

The dose of the compound (I) or a pharmaceutically acceptable salt thereof in the hypotensive agent may vary depending on the administration routes, the age, body weight or condition of patients and the kinds of diseases to be treated. In general, the preferred dose of the compound (I) or a salt thereof is usually in the range of 1 to 200 mg/kg/day, especially 3 to 100 mg/kg/day.

The phenoxyacetic acid derivatives (I) can be prepared, for example, by reacting a phenol derivative of the formula:

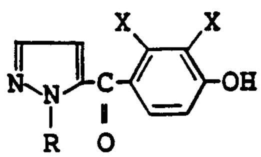

wherein R and X are the same as defined above, with an acetic acid compound of the formula: Y-$CH_2COOR^1$, wherein Y is a halogen atom and $R^1$ is a lower alkyl group, to give a phenoxyacetic acid ester derivative of the formula:

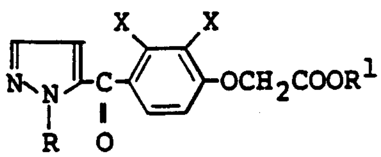

wherein $R^1$, R and X are the same as defined above, and subsequently by subjecting the said phenoxyacetic acid ester derivative to hydrolysis by the conventional method.

This invention is illustrated by the following Experiments and Preparations but should not be construed to be limited thereto.

## Experiments

| [Test compounds] | |
|---|---|
| Test Comp. No. | Chemical Name |
| (1) | [2,3-dichloro-4-(1-ethyl-5-pyrazolylcarbonyl)phenoxy]acetic acid |
| (2) | [2,3-dichloro-4-(1-ethoxymethyl-5-pyrazolylcarbonyl)phenoxy]acetic acid |

### Experiment 1

Hypotensive activity in SHR by single administration of test compound

[Method and Results]

A suspension of a test compound in water containing a small amount of Tween 80 was administered orally in a volume of 1 ml/100 g of rat's weight (a dose of the test compound: 200 mg/kg) to SHR (about 40 weeks old, 4 to 6 rats/group) fasted overnight. The blood pressure before and after administration of the test compound were measured noninvasively by the tail-cuff method, and estimated the rate of the change of the blood pressure 2 hours after administration of the test compound against the blood pressure before administration of the test compound. In both of test compound No. (1) and No. (2), the rate of change of the blood pressure was about -13 %.

Experiment 2

Antihypertensive activity on progressive hypertention in DOCA-salt rats

[Method and Result]

A left kidney was taken out from male SD rats (6 weeks old, weight; about 200 g, 10 rats/group), and 1 week later, silicon resin containing 150 mg/kg of deoxycorticosterone acetic acid (DOCA) was embedded in subcutaneous tissue at the back of each rat under ether anesthesia. After operation, the rats were provided with water containing 1 % sodium chloride and 0.2 % potassium chloride as drinking water in order to produce DOCA-salt rats. From the next day after operation of embedding the silicon resin containing DOCA, a suspension of the test compound (I) in water containing a small amount of Tween 80 was administered orally in a volume of 10 ml/kg (a dose of the test compound: 50 mg/kg/day) once a day for 4 weeks in the test compound-administered group. On the other hand, the same amount of water containing a small amount of the Tween 80 was administered in the control group instead of the suspension of the test compound (1). The systolic blood pressure of rats in the both groups was measured once a week until the fourth week by the tail-cuff method. The results are shown in the following Table 1.

Table 1

| | Systolic blood pressure (mmHg) | | | | |
|---|---|---|---|---|---|
| | Time after administration (days) | | | | |
| | 0 | 7 | 14 | 21 | 28 |
| Test compound-administered group | 121.6 | 140.3 | 165.0* | 182.0* | 193.3* |
| Control group | 121.4 | 146.2 | 178.1 | 200.2 | 213.9 |

(*: P<0.05)

As is shown in Table 1, the increase of blood pressure of rats in the test compound-administered group was inhibited significantly from the second week after administration as compared with control group.

Experiment 3

Antihypertensive activity in SHR

[Method and Results]

A suspension of the test compound (1) in water containing a small amount of Tween 80 was administered orally in a volume of 10 ml/kg (a dose of the test compound: 100 mg/kg/day) once a day for 4 weeks to SHR (5 weeks old, weight; about 100 g, 10 rats/group) in the test compound-administered group. On the other hand, the same amount of water containing a small amount of Tween 80 was administered to the rats in control group instead of the suspension of the test compound (1). The systolic blood pressure of rats of the both groups was measured once a week until the fifth week by the tail-cuff method. The results are shown in the following Table 2.

Table 2

| | Systolic blood pressure (mmHg) | | | | | |
|---|---|---|---|---|---|---|
| | Time after administration (days) | | | | | |
| | 0 | 7 | 14 | 21 | 28 | 35 |
| Test comp.-adm. group | 131.2 | ** 141.0 | ** 161.9 | ** 175.4 | ** 185.2 | ** 198.4 |
| Control group | 130.3 | 152.6 | 172.5 | 189.2 | 200.8 | 208.8 |

(**: P<0.01)

As is shown in Table 2, the increase of blood pressure of rats in the test compound-administered group was inhibited significantly from the first week after administration as compared with control group.

Experiment 4

Antihypertensive activity in SHR being in the established hypertension condition

[Method and Results]

Using the SHR being in the established hypertension (established-SHR, 18 weeks old, weight; 300 to 375 g, 8 rats/group), the same procedures were carried out as Experiment 3 except that the period of administration of the test compound (1) was 3 weeks, and the systolic blood pressure was measured until the third week after administration. The results are shown in the following Table 3.

Table 3

| | Systolic blood pressure (mmHg) | | | |
|---|---|---|---|---|
| | Time after administration (days) | | | |
| | 0 | 7 | 14 | 21 |
| Test compound-administered group | 207.3 | * 189.6 | * 201.8 | * 198.6 |
| Control group | 208.8 | 206.8 | 209.5 | 210.5 |

(*: P<0.05)

As is shown in Table 3, the blood pressure of rats in the test compound-administered group was decreased significantly from the first week after administration in comparison with the blood pressure of the rats in control group.

Experiment 5

Antihypertensive activity in the DOCA-salt rats being in the established hypertension condition

[Method and Results]

The DOCA-salt loaded rats being in the stable hypertensive condition were obtained by feeding the DOCA-salt loaded rats used in Experiment 2 with tap water instead of aqueous salt solution for 6 weeks. Using these established DOCA-salt hypertensive rats (8 rats/group), the same experiments were carried out as Experiment 3 except that the dose of the test compound (1) was 50 mg/kg/day, the period of administration was 3 weeks, and the systolic blood pressure was measured until the fourth week after administration. The results are shown in the following Table 4.

Table 4

| | Systolic blood pressure (mmHg) | | | | |
|---|---|---|---|---|---|
| | Time after administration (days) | | | | |
| | 0 | 7 | 14 | 21 | 28 |
| Test compound-administered group | 171.1 | 167.4 | 168.1* | 172 0* | 170.7* |
| Control group | 175.6 | 181.0 | 193.6 | 189.5 | 191.8 |

(*: P<0.05)

As is shown in Table 4, the blood pressure of rats in the test compound-administered group was decreased significantly from the second week after administration in comparison with the blood pressure of the rats in control group.

Preparation 1

(1) 2,3-Dichloro-4-(1-ethyl-5-pyrazolylcarbonyl)phenol (2.12 g), ethyl bromoacetate (1.61 g), and potassium carbonate (2.36 g) are added into acetone (80 ml) and the mixture is refluxed for 1.5 hours. The reaction mixture is filtered and the solvent is distilled off from the filtrate. The residue is dissolved in benzene and treated with charcoal and further the solvent is distilled off therefrom. The residue is crystallized from isopropyl ether to give [2,3-dichloro-4-(1-ethyl-5-pyrazolylcarbonyl)phenoxy]acetic acid ethyl ester (2.20 g) as colorless crystals, m.p. 79 - 81 ° C.

(2) To a suspension of the above product (1.6 g) in ethanol (25 ml) is added 10 % aqueous sodium hydroxide solution (15 ml) and the mixture is stirred at room temperature for 1 hour. Ethanol is distilled off from the reaction solution, and the mixture is adjusted to pH 1 - 2 with 10 % hydrochloric acid. The precipitated crystals are collected by filtration. The said crystals are washed successively with water and isopropyl alcohol and subsequently dried to give [2,3-dichloro-4-(1-ethyl-5-pyrazolylcarbonyl)-phenoxyacetic acid (1.40 g), m.p. 203 - 204 ° C.

Mass (m/e): 342 ($M^+$)

Sodium salt monohydrate thereof, m.p. 243.5 ° - 245.5 ° C.

Preparations 2 - 6

The compounds in the following Table 5 are obtained by treating the corresponding starting materials in the same manner as Example 1.

Table 5

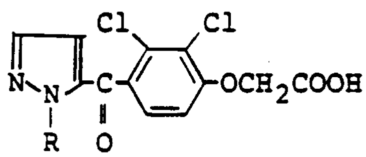

(I-a)

| Prep. No. | R | Physical properties |
|---|---|---|
| 2 | $-CH_2OCH_3$ | m.p. 179 - 180.5°C<br>Mass (m/e): 358 ($M^+$) |
| 3 | $-CH(CH_3)_2$ | m.p. 223 - 225°C<br>Mass (m/e): 356 ($M^+$) |
| 4 | $-(CH_2)_5CH_3$ | m.p. 114 - 115°C<br>Mass (m/e): 398 ($M^+$) |
| 5 | $-CH_3$ | m.p. 230 - 231°C<br>Mass (m/e): 328 ($M^+$) |
| 6 | $-CH_2OC_2H_5$ | m.p. 148 - 149°C<br>Mass (m/e): 372 ($M^+$) |

## Claims

1. Use of a phenoxyacetic acid derivative of the formula:

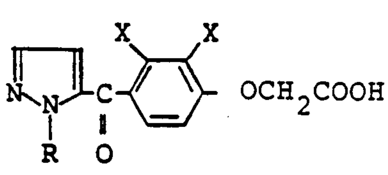

(I)

wherein R is a $C_1$-$C_6$-alkyl group or a $C_1$-$C_6$-alkoxy-substituted $C_1$-$C_6$-alkyl group and X is a halogen atom, or of a pharmaceutically acceptable salt thereof in admixture with a pharmaceutically acceptable carrier or diluent, for the manufacture of a hypotensive agent.

2. The use according to claim 1, wherein as the active ingredient, the compound of the formula (I), wherein X is a chlorine atom, or a pharmaceutically acceptable salt thereof is used.

3. The use according to claim 2, wherein as the active ingredient, the compound of the formula (I), wherein R is an alkyl group of 1 to 4 carbon atoms or an alkyl group of 1 to 3 carbon atoms substituted

by an alkoxy group of 1 to 3 carbon atoms, or a pharmaceutically acceptable salt thereof is used.

**4.** The use according to claim 2, wherein as the active ingredient, the compound of the formula (I), wherein R is an ethyl group or an ethoxymethyl group, or a pharmaceutically acceptable salt thereof is used.

**5.** The use according to claim 1, wherein as the active ingredient, [2,3-dichloro-4-(1-ethoxymethyl-5-pyrazolylcarbonyl)phenoxy]acetic acid or a pharmaceutically acceptable salt thereof is used.

**6.** The use according to claim 1, wherein as the active ingredient, [2,3-dichloro-4-(1-ethyl-5-pyrazolylcarbonyl)phenoxy]acetic acid or a pharmaceutically acceptable salt thereof is used.

**Patentansprüche**

**1.** Verwendung eines Phenoxyessigsäurederivats der Formel:

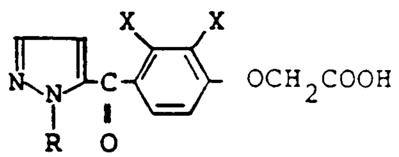

(I)

worin R eine $C_1$-$C_6$-Alkylgruppe oder eine $C_1$-$C_6$-Alkoxy-substituierte $C_1$-$C_6$-Alkylgruppe bedeutet, und X ein Halogenatom bedeutet, oder eines pharmazeutisch annehmbaren Salzes davon, im Gemisch mit einem pharmazeutisch annehmbaren Träger oder Verdünnungsmittel zur Herstellung eines blutdrucksenkenden Mittels.

**2.** Verwendung nach Anspruch 1, dadurch **gekennzeichnet**, daß als Wirkstoff die Verbindung der Formel I, worin X ein Chloratom bedeutet, oder ein pharmazeutisch annehmbares Salz davon verwendet wird.

**3.** Verwendung nach Anspruch 2, dadurch **gekennzeichnet**, daß als Wirkstoff die Verbindung der Formel I, worin R eine Alkylgruppe mit 1 bis 4 Kohlenstoffatomen oder eine Alkylgruppe mit 1 bis 3 Kohlenstoffatomen, welche durch eine Alkoxygruppe mit 1 bis 3 Kohlenstoffatomen substituiert ist, bedeutet, oder ein pharmazeutisch annehmbares Salz davon verwendet wird.

**4.** Verwendung nach Anspruch 2, dadurch **gekennzeichnet**, daß als Wirkstoff die Verbindung der Formel I, worin R eine Ethylgruppe oder eine Ethoxymethylgruppe bedeutet, oder ein pharmazeutisch annehmbares Salz davon verwendet wird.

**5.** Verwendung nach Anspruch 1, dadurch **gekennzeichnet**, daß als Wirkstoff [2,3-Dichlor-4-(1-ethoxymethyl)-5-pyrazolylcarbonyl)phenoxy]essigsäure oder ein pharmazeutisch annehmbares Salz davon verwendet wird.

**6.** Verwendung nach Anspruch 1, dadurch **gekennzeichnet**, daß als Wirkstoff [2,3-Dichlor-4-(1-ethyl-5-pyrazolylcarbonyl)phenoxy]essigsäure oder ein pharmazeutisch annehmbares Salz davon verwendet wird.

## Revendications

1. Utilisation d'un dérivé d'acide phénoxyacétique de formule :

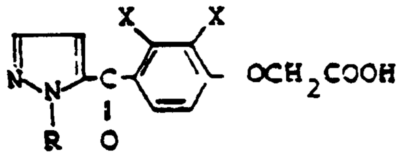

(I)

dans laquelle R est un groupe $C_1$-$C_6$-alkyle ou un groupe $C_1$-$C_6$-alkyle à substitution $C_1$-$C_6$-alcoxy et X est un atome d'halogène, ou d'un sel pharmaceutiquement acceptable dudit dérivé en mélange avec un support ou diluant pharmaceutiquement acceptable, pour la fabrication d'un agent hypotenseur.

2. L'utilisation selon la revendication 1, selon laquelle, comme ingrédient actif, le composé de formule (I), dans laquelle X est un atome de chlore, ou un sel pharmaceutiquement acceptable de ce composé est utilisé.

3. L'utilisation selon la revendication 2, selon laquelle comme ingrédient actif, le composé de formule (I), dans laquelle R est un groupe alkyle de 1 à 4 atomes de carbone ou un groupe alkyle de 1 à 3 atomes de carbone substitué par un groupe alcoxy de 1 à 3 atomes de carbone, ou un sel pharmaceutiquement acceptable de ce composé est utilisé.

4. L'utilisation selon la revendication 2, selon laquelle comme ingrédient actif, le composé de formule (I), dans laquelle R est un groupe éthyle ou un groupe éthoxyméthyle, ou un sel pharmaceutiquement acceptable de ce composé est utilisé.

5. L'utilisation selon la revendication 1, selon laquelle comme ingrédient actif, l'acide [2,3-dichloro-4-(1-éthoxyméthyl-5-pyrazolycarbonyl)phénoxy]acétique ou un sel pharmaceutiquement acceptable de cet acide est utilisé.

6. L'utilisation selon la revendication 1, selon laquelle comme ingrédient actif, l'acide [2,3-dichloro-4-(1-éthyl-5-pyrazolylcarbonyl)phénoxy]acétique ou un sel pharmaceutique acceptable de cet acide est utilisé.